Europäisches Patentamt

European Patent Office

Office européen des brevets

(11) Publication number: **0 397 999 A1**

## EUROPEAN PATENT APPLICATION

(21) Application number: **90105830.5**

(22) Date of filing: **27.03.90**

(51) Int. Cl.⁵: **A61F 13/04, B63C 11/04**

(30) Priority: **19.05.89 US 354648**
**22.09.89 US 411359**

(43) Date of publication of application:
**22.11.90 Bulletin 90/47**

(84) Designated Contracting States:
**DE FR GB SE**

(71) Applicant: **W.L. GORE & ASSOCIATES, INC.**
**551 Paper Mill Road P.O. Box 9206**
**Newark, DE 19714(US)**

(72) Inventor: **Keller, Mary Lou**
**8 Clarion Court**
**Newark, Delaware 19714(US)**

(74) Representative: **Klunker . Schmitt-Nilson .**
**Hirsch**
**Winzererstrasse 106**
**D-8000 München 40(DE)**

(54) **Protective padding.**

(57) A protective padding, characterized by liquid-water impermeability and water vapor-permeability is provided. The padding comprises a middle layer of padding (12) and top and bottom (14) layers of nonporous breathable films. The top and bottom layers may alternatively be comprised of one layer of nonporous film (17) and one layer of porous film (15). The padding is useful in diverse applications such as the underpadding for orthopedic immobilizing devices and as insulation capable of withstanding water immersion.

Fig. 1

EP 0 397 999 A1

## FIELD OF THE INVENTION

This invention relates to a protective padding that provides cushioning and insulation, and is also liquid water-impermeable and water vapor-permeable. The padding is particularly useful underneath orthopedic casts and braces.

## BACKGROUND OF THE INVENTION

Materials used for padding have included natural materials such as cotton, wool or down; and synthetics such as polyester fiberfill and foams. The major functions of padding are cushioning, filling, and insulation. Medical uses for paddings include applying or relieving pressure and absorption of excess moisture. Currently available paddings lose some of their functionality when immersed in water. Water can cause padding to compress or to shift due to weight gain. It also alters the insulating properties of most paddings and can cause extreme discomfort. In addition, a water saturated padding is no longer useful for transfer of fluids away from the skin. This loss of function is especially critical when the padding is to be worn by a person for extended periods. Prolonged wetness next to skin causes skin breakdown and possible infection sites. It is therefore desirable to make a padding which will not be adversely affected by exposure to water. It is also desirable to make a padding that will allow the transfer of vapors so that perspiration and other body fluids can escape.

In the past, several methods have been used to overcome some of the drawbacks of padding. Quilting has been used to prevent padding from shifting, while maintaining the high loft and bulkiness desirable in padding. Bonding padding to a fabric backing also prevents shifting. Fiberfill consists of crimped fibers to help maintain fluffiness and air space, even upon exposure to water. Hollow fibers also contain air space which add insulating value. Synthetic fibers are generally less absorbent than natural ones and have been used to wick fluids away from the skin into more absorbent backings. However, fluid saturation results in loss of function.

Padding is used under immobilizing orthopedic casts or braces. Traditional cast underpadding consists of cotton or polyester wraps in 2" to 6" widths. Care is taken when applying the underpadding to avoid folds or creases which can cause pressure sores to the skin. The immobilizing material applied on top of this padding is usually made of plaster of paris or polyurethane coated fiberglass. Cast wearers are usually told not to immerse the cast in water. If a traditionally padded polyurethane cast is immersed, the padding remains wet for many hours, and the result is skin maceration. In some cases, this maceration can lead to skin breakdown and infection by bacteria or fungi.

Cast material manufacturers caution against water immersion and state that if a cast is wet it should be completely dried with a hair dryer. This drying process can take several hours and patient compliance is generally low. Some cast wearers use plastic bags with rubber bands around the end to keep water away from the cast while showering. Water often enters the bag and wets the padding which causes discomfort to the wearer. As a result most cast wearers do not get their cast wet and spend the time they are in a cast without a normal shower or bath. In addition, cast wearers cannot swim or use any form of hydrotherapy.

It is widely recognized that paddings should be "breathable" to be comfortable, i.e. should allow water vapor to pass through. However, it is not necessary that air pass through the padding for it to be comfortabe, only that water vapor from perspiration or other sources be transmitted from the skin outwards through the padding. The terms "breathability" and "the ability to transport interior moisture vapor to the external environment" are used interchangeably herein.

In addition, there are instances where thermal insulation in apparel should be kept completely dry. To date, insulations are protected only from one side. However, when perspiration is heavy, or there is risk of immersion, a completely waterproof insulation is desirable.

It would be desirable to provide a padding for use as herein above described that is liquid water-impermeable and water vapor-permeable.

## SUMMARY OF THE INVENTION

This invention provides a liquid water-impermeable and water vapor-permeable protective padding material. The material comprises a middle layer of padding; and a top and bottom layer, each comprising a water-impermeable and water vapor-permeable film, with at least one said film being nonporous.

## BRIEF DESCRIPTION OF THE DRAWINGS

Figure 1 shows a cross section of the protective padding.

Figure 2 shows a top view of a quilted protective padding.

Figure 3 shows a cross section of the padding of Figure 2 taken along lines 3-3 of Figure 2.

Figure 4 shows a cross section of the padding described in Example 1.

Figure 5 shows the protective padding of Figure 1 in place underneath a cast around a human leg.

## DESCRIPTION OF THE INVENTION

As previously described, this invention provides a liquid water-impermeable and water vapor-permeable protective padding material. The protective padding consists of standard padding materials such as cotton or fiberfill. The protective outer layers can be a polymeric film or laminate that encapsulates the padding. One or both the outer layers provide a waterproof, "breathable" nonporous barrier, while the inner layer of padding provides a cushioning and insulating filler. By nonporous is meant that while the layer is breathable, the layer has no pores or else pores are filled to close them off. A number of different materials are suitable for the outer layer. The outer layers are made of a waterproof, water vapor-permeable material. Nonporous films suitable for this application include polyurethane coatings with or without a porous substrate, polyester as detailed in U.S. Patent 4,493,870 and polyether polyurethanes.

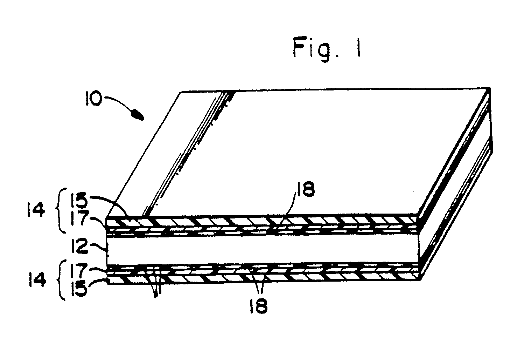

Referring to Figure 1, laminate 10 comprises middle layer of padding 12, such as cotton or fiberfill or a woven, knitted or non-woven material. Bonded to each side of middle layer 12 is a layer 14 containing a sheet 15 of water-impermeable, water vapor-impermeable material. Layers 14 are bonded to layer 12 by means of an adhesive 18 as shown between the middle layer 12 and the top and bottom layers 14. One layer 15 comprises a porous layer and the other layer comprises a nonporous layer. A hydrophilic layer 17 as disclosed in U.S. Patent 4,194,041 is preferred for certain uses. However, the layers 15 may be bonded directly to the middle layer 12 by adhesive 18. Adhesive layer 18 can be a reactive polyurethane prepolymer.

In addition, it may also be desirable to divide the protective padding into smaller units or cells through quilting or embossing. This is useful not only to prevent shifting but also to protect each cell from water which may enter through a hole or defect in adjacent cells. This allows the protective padding to be cut or trimmed without violating the integrity of the whole pad.

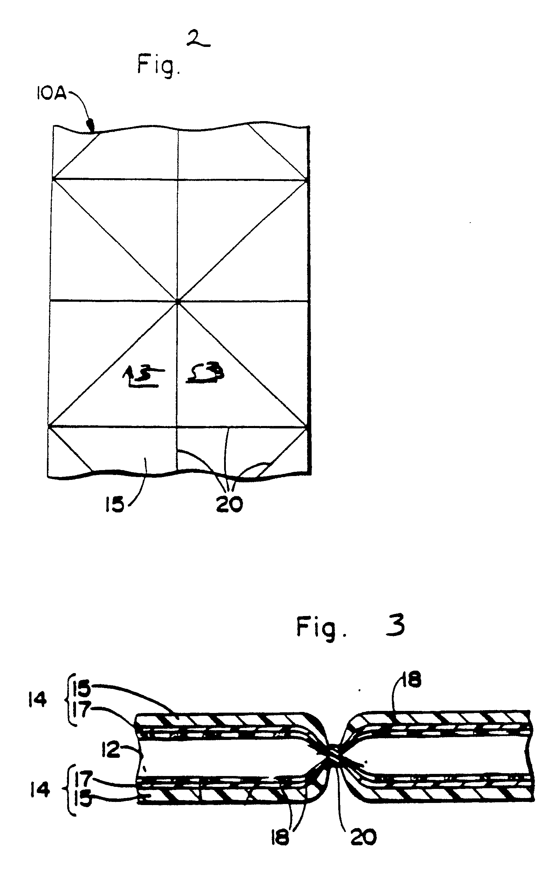

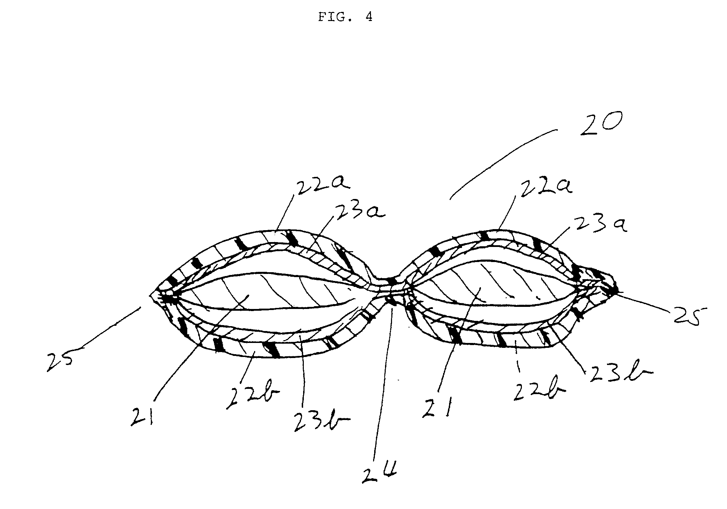

Figure 2 is a top plan view of a quilted embodiment 10A of padding for use in an orthopedic or a clothing garment, wherein the quilted pattern is in the form of triangles bounded by quilt lines 20 and Figure 3 shows a cross-sectional view taken along line 3-3 of Figure 2.

It may also be desirable to bond the film to a supportive fabric.

The protective padding can be manufactured by sealing the edges of the outer layers, i.e. around the periphery to completely encompass the middle padding. This sealing can be done in many ways, for example, using heat, ultrasonic welding, or adhesives. Quilting can be done if thermoplastic fibers are used as the padding filler and heat or ultrasound can be used to seal the edges and to create individual cells.

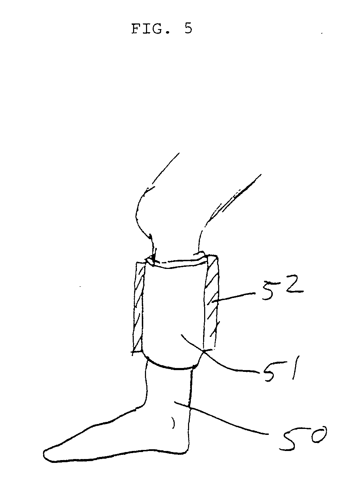

Figure 4 depicts, a cross section of the invention described in Example 1. The protective padding 20 comprises a middle layer of padding material 21 which is polyester and top and bottom outer protective layers made of a sheet 22 of breathable nonporous material (which is a polyurethane). The top and bottom outer protective layers are bonded to the middle layer 21 at quilt bond point 24 by simply thermally sealing the materials together, and at outer periphery bond seams 25 and 26. The coated water-impermeable, water vapor-permeable top and bottom outer protective layers and the middle padding layer are not bonded or attached to each other at any other points.

The protective padding operates under several principles. Firstly, the outer layers do not allow liquid water to pass to the inner layers. Therefore, water will drain out or will pass through the film only after it has been vaporized. Additionally, the body heat of the wearer causes this evaporation process to occur and creates a one way path for water vapor. If the inner padding becomes moist, the moisture is still directed outwards through the cast, not back towards the person. Therefore, padding encapsulated by the aforementioned outer layer films does not experience the same water weight gain that unprotected padding does, and the wearer does not have to wait as long to experience dryness. The skin surface dries much faster with the protective padding than with unprotected padding. Perspiration is allowed to pass through the padding without moisture remaining on the skin. Traditional padding remains damp for many hours, but the protective padding dries quickly. This provides the wearer with a great deal of comfort.

Many applications can be envisioned for such a protective padding. In addition to outstanding insulation, it provides for fast skin surface drying. Any use which requires the skin to be subjected to wet or damp conditions for prolonged periods would benefit from a padding which transfers water vapor away from the skin. Of special interest is the use of this protective padding to protect persons required to wear an orthopedic cast or brace. There are many benefits of using a waterproof, breathable cast underpadding. Such a padding allows cast or brace wearers to shower or swim while immobilized without special precautions or drying procedures. This is advantageous not only for hygenic purposes, but also for therapeutic modalities. Odor and itching is reduced for cast patients if washing is allowed on a normal schedule. Cast wearers could engage in activities which may cause perfuse perspiration without the discomfort of wet padding. Use of hydrotherapy could aid in the healing process, as it does in some other injuries which do not require immobilization.

The protective padding can be applied in a wrap formation similar to traditional cast underpaddings, or it can be configured to fit around the damaged limb in a single layer. The thinness of the film is essential to avoid bulky folds which can cause pressure sores on the skin.

Referring to Figure 5, protective padding 51 of the invention is placed around leg 50 and orthopedic cast 52 (shown cut away) is placed around padding 51.

In addition, many athletes require the use of supportive braces to continue their training. Use of the padding of this invention prevents perspiration from being trapped next to the skin following physical exertion. Alternatively, the padding can be incorporated directly into the brace, perhaps with a fabric laminated to the film.

In addition, this padding can be used in manufacturing apparel and outdoor gear such as tents, sleeping bags, etc. to provide a product which can withstand water immersion.

## EXAMPLES

### Example 1

A layer of breathable nonporous polyurethane film 9 inches wide and 12 inches long was laid down on a flat rubber mat. A 4 inch wide strip of polyester cast underpadding was laid on top of the polyurethane film and the film was folded over the underpadding. A layer of Kapton® film was laid over top of the polyurethane film to allow a high temperature soldering iron to seal around the padding, making a seam down the open edge, enclosing the underpadding within the polyurethane film. The excess nonporous film was trimmed, excess air was voided, and the ends were sealed. The Kapton film was removed. The resultant padding was worn on top of the skin for several hours. No discomfort was noted.

### Example 2

A layer of breathable polyurethane film 4 inches wide and 6 inches long was laid down on a flat rubber mat. A 2 inch wide strip of polyester underpadding was laid on top of the polyurethane film. A layer of porous polytetrafluoroethylene (PTFE) coated with a hydrophilic impregnant 4 inches wide and 6 inches long was laid on top of the padding and the polyurethane film, with the hydrophilic impregnant towards the padding. A high temperature soldering iron was used to seal around the padding, making a seam down the open edge, enclosing the underpadding within the porous and nonporous films. The excess film was trimmed, excess air was voided, and the ends were sealed. The resultant padding was worn on top of the skin for several hours. No discomfort was noted.

## Claims

1. A protective padding, characterized by liquid-water impermeability and water vapor-permeability, said padding comprising:
   (a) a middle layer of padding, and
   (b) a top and bottom layer each comprising water-impermeable and moisture vapor-permeable film, with at least one of said films being nonporous.

2. A protective padding material of Claim 1, wherein the top and bottom layers are both nonporous.

3. A protective padding material as defined in Claim 1, where the top and bottom layers are polyurethane film.

4. A protective padding material as defined in anyone of the claims 1, 2 or 3 wherein at least one of the top and bottom films is bonded to a supportive fabric.

5. A protective padding as defined in anyone of the claims 2 or 3, where the top and bottom layers are bonded at the edges to encompass the padding.

6. A protective padding as defined in at least one of the claims 1 to 5, where the inner layer is a thermoplastic padding which can be secured within the top and bottom layer by heat or ultrasonic welding.

7. A protective padding as defined in claim 5, where an additional immobilizing layer is applied on top of the protective padding.

8. A protective padding as defined in claim 7, where the additional immobilizing layer is a water compatible orthopedic cast material.

9. A protective padding as defined in at least one of the claims 1 to 8, where the additional immobilizing layer is an orthopedic brace.

10. A protective padding as defined in at least one of the preceeding claims, where the padding is incorporated into a garment or outdoor gear.

11. A protective padding of claim 1 wherein the top and bottom layers are joined together in discrete quilt patterns by heat fusing the middle layer only along the peripheral boundaries of the quilt pattern compressing the outer layers together.

# Fig. 1

Fig. 2

10A

15

20

Fig. 3

14 { 15
      17

12

14 { 17
      15

18

18    20

FIG. 4

FIG. 5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 296 364 (L.J. RAUTENBERG et al.)<br>* Page 3, lines 25-29; page 4, lines 40-44; page 8, lines 21-35; figure 2A * | 1-5,10 | A 61 F 13/04<br>B 63 C 11/04 |
| Y |  | 6-9,11 | |
| Y | EP-A-0 289 681 (W.L. GORE & ASSOCIATES)<br>* Column 2, lines 2-37; claim 4 * | 7-8 | |
| Y | US-A-4 788 972 (A.O.V. DEBUSK)<br>* Column 1, line 62 - column 2, line 12; figure 3 * | 9 | |
| Y | FR-A-2 080 019 (FABRIQUE DE SOIERIES ANDRE & SERGE FERRARI)<br>* Page 1, lines 1-4,20-26; page 2, line 40 - page 3, line 6; page 3, lines 14-18; figures 1,3 * | 6,11 | |
| A |  | 5,9 | TECHNICAL FIELDS SEARCHED (Int. Cl.5) |
| A | US-A-4 194 041 (R.W. GORE & S.B. ALLEN, Jr.)<br>* Column 3, line 23 - column 4, line 17; column 5, lines 39-41; column 8, lines 7-8; claim 10 * | 1-4,9 | A 61 F<br>A 41 D |

The present search report has been drawn up for all claims

| Place of search | Date of completion of the search | Examiner |
|---|---|---|
| THE HAGUE | 03-08-1990 | NICE P.R. |

EPO FORM 1503 03.82 (P0401)